# EUROPÄISCHE PATENTANMELDUNG

(11) **EP 4 477 640 A1**
(43) Veröffentlichungstag der Anmeldung: **18.12.2024**
(21) Anmeldenummer: 23179204.5
(22) Anmeldetag: 14.06.2023
(51) Int. Cl.: C07C 7/04, C07C 45/50, C07C 45/82, C07C 11/02

(54) **VERFAHREN ZUR HYDROFORMYLIERUNG VON DI-ISOBUTEN MIT VORHERIGER DESTILLATION**

(71) Anmelder: Evonik Oxeno GmbH & Co. KG, 45772 Marl (DE)
(72) Erfinder: KUCMIERCZYK, Peter, 44628 Herne (DE); FRANKE, Robert, 45772 Marl (DE); SALE, Anna Chiara, 45657 Recklinghausen (DE); FRIDAG, Dirk, 45721 Haltern am See (DE); MARKOVIC, Ana, 45721 Haltern am See (DE); RIX, Armin Matthias, 45770 Marl (DE); TERMÜHLEN, Maren, 44135 Dortmund (DE); HARDING, Laura-Selin, 46286 Dorsten (DE); METTERNICH, Jan Benedikt, 45657 Recklinghausen (DE)
(74) Vertreter: Evonik Patent Association

(57) **Zusammenfassung**

Gegenstand der Erfindung ist ein Verfahren zur Hydroformylierung von Di-isobuten, bei dem der eingesetzte Di-isobutenstrom vor der Hydroformylierung einer Destillation unterworfen wird, um 2,4,4-Trimethylpent-1-en in dem zu hydroformylierenden Strom anzureichern. Die Hydroformylierung wird mit Synthesegas in Gegenwart eines homogenen Katalysatorsystems, welches zumindest Co oder Rh und optional einen phosphorhaltigen Liganden umfasst, durchgeführt.

## Beschreibung

Die vorliegende Erfindung betrifft ein Verfahren zur Hydroformylierung von Di-isobuten, bei dem der eingesetzte Di-isobutenstrom vor der Hydroformylierung einer Destillation unterworfen wird, um 2,4,4-Trimethylpent-1-en in dem zu hydroformylierenden Strom anzureichern. Die Hydroformylierung wird mit Synthesegas in Gegenwart eines homogenen Katalysatorsystems, welches zumindest Co oder Rh und optional einen phosphorhaltigen Liganden umfasst, durchgeführt.

Di-isobuten ist ein technisch relevantes Erzeugnis, welches durch Dimerisierung von Isobuten gewonnen wird. Di-isobuten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 von etwa 78:22 bis 81 : 19 (Gleichgewichtsverteilung). Diese Mischung kann unter anderem in Carbonylierungsverfahren zu höherwertigen Produkten umgesetzt werden. Dabei weist speziell in Carbonylierungsverfahren, wie der Methoxycarbonylierung (Reaktionsprodukt ist hier der 3,5,5-Trimethylhesansäuremethylester) oder der Hydroformylierung (Reaktionsprodukt ist hier der 3,5,5-Trimethylhexanal) das innenständige Olefin TMP2 eine deutlich geringere Reaktivität als das endständige Olefin TMP1 auf. Oftmals können Reaktionsbedingungen oder Verweilzeiten bei diesen Carbonylierungsreaktionen aufgrund der mangelnden Stabilität der Katalysatoren oder aufgrund ökonomischer Faktoren (z. B. der Reaktorgröße) nicht dahingehend angepasst werden, dass das endständige TMP2 vollständig abreagiert.

Die Aufgabe der vorliegenden Erfindung war deshalb ein Verfahren zur Hydroformylierung von Strömen, welches die vorgenannten Probleme nicht aufweist. Insbesondere sollte die Hydroformylierung mit Di-isobutenströmen, die einen hohen Anteil an 2,4,4-Trimethylpent-1-en aufweisen, durchgeführt werden.

Diese Aufgabe konnte durch das erfindungsgemäße Verfahren nach dem unabhängigen Anspruch 1 gelöst werden. Bevorzugte Ausführungsformen sind in den abhängigen Ansprüchen angegeben.

Beschrieben wird vorliegend ein Verfahren zur Hydroformylierung von Di-isobuten, wobei das Verfahren zumindest die folgenden Schritte umfasst:
a. Zuführen eines Di-isobutenstroms, der 2,4,4-Trimethylpent-2-en und 2,4,4-Trimethylpent-1-en enthält, zu mindestens einer Destillationskolonne und Auftrennen des Di-isobutenstroms in mindestens einen gegenüber dem eingesetzten Di-isobutenstrom an 2,4,4-Trimethylpent-1-en angereicherten Kopfstrom, der mehr als 85 Gew.-% an 2,4,4-Trimethylpent-1-en enthält, und einen Reststrom, der gegenüber dem eingesetzten Di-isobutenstrom an 2,4,4-Trimethylpent-1-en abgereichert ist;
b. Zuführen des Kopfstroms zur Hydroformylierung, wodurch 2,4,4-Trimethylpent-2-en und 2,4,4-Trimethylpent-1-en mit Synthesegas in Gegenwart eines homogenen Katalysatorsystems, welches zumindest Co oder Rh und optional einen phosphorhaltigen Liganden umfasst, in einer Reaktionszone unter Erhalt eines flüssigen Produktgemisches umgesetzt werden, wobei das flüssige Produktgemisch zumindest die durch die Hydroformylierung gebildeten Aldehyde, das homogene Katalysatorsystem und nicht umgesetztes 2,4,4-Trimethylpent-2-en und 2,4,4-Trimethylpent-1-en umfasst;
c. Abtrennen des homogenen Katalysatorsystems aus dem flüssigen Produktgemisch unter Erhalt eines Rohproduktgemisches, welches zumindest die durch die Hydroformylierung gebildeten Aldehyde und nicht umgesetztes 2,4,4-Trimethylpent-2-en und 2,4,4-Trimethylpent-1-en umfasst;
d. Destillative Aufarbeitung des Rohproduktgemisches in mindestens einer Destillationskolonne zur Abtrennung von nicht umgesetzten 2,4,4-Trimethylpent-2-en und nicht umgesetzten 2,4,4-Trimethylpent-1-en, wobei das nicht umgesetzte 2,4,4-Trimethylpent-2-en und 2,4,4-Trimethylpent-1-en abgetrennt und zur Hydroformylierung in Schritt b zurückgeführt wird.

Ein Vorteil des Verfahrens ist die Destillation des Di-isobutenstroms vor der Hydroformylierung, weil dadurch der Anteil an 2,4,4-Trimethylpent-1-en im Kopfstrom der mindestens einen Destillationskolonne gegenüber dem eingesetzten Strom gesteigert werden kann. So ist es möglich Anteile von mehr als 85 Gew-%, vorzugsweise mehr als 90 Gew.-%, besonders bevorzugt mehr als 95 Gew.-% an 2,4,4-Trimethylpent-1-en im Strom zu erreichen, was für die nachgeschaltete Hydroformylierung aus den genannten Gründen (höhere Reaktivität von 2,4,4-Trimethylpent-1-en) vorteilhaft ist.

Schritt a des erfindungsgemäßen Verfahrens betrifft die Destillation eines Di-isobutenstroms, welcher 2,4,4-Trimethylpent-2-en und 2,4,4-Trimethylpent-1-en enthält. Der Di-isobutenstrom wird dabei in mindestens einen gegenüber dem eingesetzten Di-isobutenstrom an 2,4,4-Trimethylpent-1-en angereicherten Kopfstrom, der vorzugsweise mehr als 85 Gew.-% an 2,4,4-Trimethylpent-1-en, weiterhin bevorzugt mehr als 90 Gew.-% an 2,4,4-Trimethylpent-1-en, besonders bevorzugt mehr als 95 Gew.-% an 2,4,4-Trimethylpent-1-en enthält, und einen Reststrom, der gegenüber dem eingesetzten Di-isobutenstrom an 2,4,4-Trimethylpent-1-en abgereichert ist, aufgetrennt.

Der in Schritt a eingesetzte Di-isobutenstrom enthält 2,4,4-Trimethylpent-2-en und 2,4,4-Trimethylpent-1-en. Derartige Ströme können Diisobutenströme sein, die mittels Dimerisierung aus Isobuten oder Isobutenhaltigen Kohlenwasserstoffgemischen hergestellt werden können, beispielsweise so wie es in EP 1 360 160 B1 offenbart wird. Weiterhin können die hier einzusetzenden Ströme als nicht reagierte Restströme bei Carbonylierungsverfahren anfallen, beispielsweise bei der Methoxycarbonylierung oder bei der Hydroformylierung. Es ist bereits erwähnt worden, dass der Einsatzstrom zur Isomerisierung und/oder zur Destillation zugeführt werden kann. Die Bezeichnung der Schritte a. und b. im vorliegenden Anspruch stellt somit keine Priorisierung oder Chronologie dar. Es versteht sich, dass beide Schritte durchgeführt werden müssen und dass die einzelnen Schritte aus dem jeweils anderen Schritt gespeist werden. Welcher Schritt also als zuerst ausgeführt angesehen wird, ist daher nebensächlich.

Die Destillation in Schritt a des erfindungsgemäßen Verfahrens wird in mindestens einer Destillationskolonne durchgeführt. Destillationskolonnen sind dem Fachmann grundsätzlich bekannt. Die Destillationseinheit der vorliegenden Erfindung umfasst vorzugsweise mindestens eine Destillationskolonne, besonders bevorzugt mindestens zwei Destillationskolonnen. Die nachfolgende Beschreibung von Merkmalen der Destillationskolonne gilt immer auch für den Fall, dass mehr als eine Destillationskolonne in der Destillationseinheit vorliegt.

Die mindestens eine Destillationskolonne weist vorzugsweise Einbauten auf, um die Trennaufgabe zu bewältigen. Entsprechende Einbauten sind dem Fachmann geläufig. Besonders geeignet sind hier regellose Packungen oder Strukturpackungen, wie sie dem Fachmann unter Handelsnamen wie MellaPak^{®}, MellapakPlus^{®}, Flexipac^{®} etc. bekannt sind. In einer bevorzugten Ausführungsform der vorliegenden Erfindung umfasst die mindestens eine Destillationskolonne mindestens 50 theoretische Trennstufen, vorzugsweise mindestens 70 theoretische Trennstufen, besonders bevorzugt mindestens 90 theoretische Trennstufen. Liegen zwei oder mehr Destillationskolonnen vor, können die Destillationskolonnen eine identische oder eine unterschiedliche Anzahl an theoretischen Trennstufen aufweisen.

Die Betriebsparameter der mindestens einen Destillationskolonne orientieren sich an der Trennaufgabe und der Gestaltung der Destillationskolonne. Im vorliegenden Verfahren ist es bevorzugt, dass die mindestens eine Destillationskolonne bei Unterdruck, besonders bevorzugt bei einem Druck von 0,2 bis 0,9 bar betrieben wird. Unterdruck liegt im Rahmen der vorliegenden Erfindung immer dann vor, wenn unterhalb des Umgebungsdrucks von ca. 1 bar, also dem an dem jeweiligen Standort vorliegenden Atmosphärendruck gearbeitet wird. Liegen zwei oder mehr Destillationskolonnen vor, können die Destillationskolonnen beim gleichen oder bei einem unterschiedlichen Druck betrieben werden.

Es ist weiterhin bevorzugt, dass die Temperatur im Sumpf der mindestens einen Destillationskolonne im Bereich von 50 bis 100 °C liegt. Liegen zwei oder mehr Destillationskolonnen vor, können die Destillationskolonnen bei der gleichen oder bei unterschiedlicher Temperatur betrieben werden.

Ein weiterer Parameter bei der Auslegung von Destillationskolonnen ist das Rücklaufverhältnis. Das Rücklaufverhältnis meint das Verhältnis Rücklauf (Rezyklat) zum Destillat (abgezogenes Kondensat, hier also der Kopfstrom K). Der Rücklauf ist also ein auskondensierter Teil des Kopfstroms, der in die Destillationskolonne zurückgeführt wird. Es ist erfindungsgemäß bevorzugt, dass das Rücklaufverhältnis der mindestens einen Destillationskolonne bei der Destillation in Schritt a. im Bereich von 5 bis 15 beträgt.

Durch die erfindungsgemäße Destillation in Schritt a fällt an der mindestens einen Destillationskolonne ein Kopfstrom an, der vorzugsweise mindestens 85 Gew.-% an 2,4,4-Trimethylpent-1-en, weiterhin bevorzugt mindestens 90 Gew.-%an 2,4,4-Trimethylpent-1-en, besonders bevorzugt mindestens 95 Gew.-% an 2,4,4-Trimethylpent-1-en enthält. Weiterhin fällt bei der Destillation ein Reststrom an, der gegenüber dem eingesetzten Di-isobutenstrom an 2,4,4-Trimethylpent-1-en abgereichert ist.

Der zusätzlich bei der Destillation in Schritt a anfallende Reststrom kann als Sumpfstrom oder als Seitenstrom an der mindestens einen Destillationskolonne abgenommen werden. Sind zwei Destillationskolonnen vorhanden, wird der Reststrom, unabhängig davon, ob als Seitenstrom oder Sumpfstrom abgenommen, in der letzten Destillationskolonne anfallen. Wird der Reststrom als Seitenstrom abgenommen kann er grundsätzlich auf gleicher Höhe oder unterhalb des Einlasses für den eingesetzten Di-isonbutenstrom angeordnet sein. Dabei versteht sich, dass Zulauf und Ablauf ausreichend beanstandet voneinander angeordnet sein müssen. Der Reststrom, unabhängig davon, ob als Seitenstrom oder Sumpfstrom abgenommen, enthält vorzugsweise 80 bis 92 Gew.-% 2,4,4-Trimethylpent-2-en.

Wird der Reststrom bei der Destillation in Schritt a als Sumpfstrom der mindestens einen Destillationskolonne abgenommen, wird vorzugsweise ein Purgestrom aus dem Reststrom abgezogen, der während der Isomerisierung entstandene Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Wird der Reststrom bei der Destillation in Schritt a als Seitenstrom der mindestens einen Destillationskolonne abgenommen, wird vorzugsweise im Sumpf der mindestens einen Destillationskolonne ein Strom anfallen, der die während der Isomerisierung entstandenen Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Destillationskolonnen werden bekanntermaßen im Sumpf beheizt, um die Trennaufgabe bewältigen zu können. Möglich ist hier eine Energiezufuhr über Heizdampf, der oft an chemischen Produktionsstandorten verfügbar ist. Dazu wird ein Teil des Sumpfes über einen Wärmetauscher (Sumpfverdampfer) geführt, dort erhitzt und dann zurück in den Sumpf der Destillationskolonne geführt. Um den Energiebedarf und/oder um die COz-Emissionen zu verringern, kann eine Wärmeintegration bei der Destillation in Schritt a. vorgesehen werden. Wärmeintegration bedeutet, dass Energie, die innerhalb des Verfahrens entsteht oder vorhanden ist, an anderer Stelle eingesetzt wird. Im vorliegenden Fall eignet sich vor allem die (freiwerdende) Kondensationsenergie, die am Kopf mindestens einer Destillationskolonne der Destillationseinheit anfällt. In einer bevorzugten Ausführungsform der vorliegenden Erfindung wird in Schritt a. also eine Wärmeintegration dergestalt durchgeführt, dass zumindest ein Teil der Kondensationsenergie am Kopf zum Erhitzen des Sumpfs genutzt wird.

Eine andere Möglichkeit zur Wärmeintegration besteht in der sogenannten Brüdenverdichtung, bei der zumindest ein Teil des Brüdens (Kopfstroms) verdichtet, also auf ein höheres Druckniveau gebracht, und optional erhitzt wird. Der so verdichtete und optional erhitzte Brüden wird zum Wärmetauscher (Sumpfverdampfer) geführt, um damit den Sumpf zu erhitzen. Hierzu wird die Kondensationswärme des Brüden genutzt.

Eine weitere Möglichkeit besteht in dem Einsatz einer Wärmepumpe. Wärmepumpen werden mit einem Arbeitsmedium, z. B. n-Butan oder Wasser betrieben. Die Kondensationsenergie wird hier also zunächst in einem Wärmetauscher auf ein Arbeitsmedium übertragen und von diesem in einem weiteren geeigneten Wärmetauscher auf den Sumpf in der Destillation. Das Arbeitsmedium wird dabei in aller Regel über einen Verdichter zum Wärmetauscher im Sumpf geführt. Es ist grundsätzlich auch möglich zweistufige oder mehrstufige Wärmepumpen einzusetzen, bei denen mehr als eine Kompressorstufe vorliegt. Bei einer zweistufigen Wärmepumpe liegt nicht nur ein Arbeitsmedium, sondern zwei Arbeitsmedien vor, wobei zwischen dem ersten und dem zweiten Arbeitsmedium ebenfalls ein Energieaustausch in einem Wärmetauscher stattfindet. Bei mehrstufigen Ausgestaltungen sind entsprechend mehr Arbeitsmedien vorhanden.

Der aus der Destillation in Schritt a erhaltene Kopfstrom wird dem nachfolgenden Schritt b der Hydroformylierung zugeführt und dort umgesetzt. Der ebenfalls aus der Destillation in Schritt a erhaltene Reststrom wird nach einer bevorzugten Ausführungsform zu einer Isomerisierung geführt, bei der 2,4,4-Trimethylpent-2-en unter Verwendung eines heterogenen Katalysators auf Basis eines Zeoliths oder eines Ionenaustauscherharzes zumindest teilweise 2,4,4-Trimethylpent-1-en isomerisiert wird. Die Isomerisierung kann also mit dem Reststrom durchgeführt, der gegenüber dem in Schritt a eingesetzten Di-isobutenstrom an 2,4,4-Trimethylpent-1-en abgereichert ist und aus der Destillation in Schritt a entnommen wird.

Die Durchführung einer Isomerisierung vor der Destillation in Schritt a hat den Vorteil, dass ein heterogenes Katalysatorsystems eingesetzt wird. Ein solches Katalysatorsystem muss nicht vom Isomerisierungsstrom abgetrennt werden und verbleibt im Reaktionsgefäß bzw. dem Reaktor. Mit den erfindungsgemäß bevorzugten Katalysatorsystemen auf Basis eines Zeoliths oder eines Ionenaustauscherharzes können zudem hohe Umsätze erzielt werden.

Die Isomerisierung kann grundsätzlich in jedem geeigneten Reaktor durchgeführt werden. Möglich ist, dass die Isomerisierung in einem einzigen Reaktor oder in mehreren parallel oder in Reihe geschalteten Reaktoren erfolgt. Möglich ist zudem die Durchführung in Chargen oder im kontinuierlichen Betrieb. Bevorzugt wird die Isomerisierung in einem oder mehreren kontinuierlich arbeitenden Reaktoren, die üblicherweise bei Feststoff/Flüssigkeits-Kontaktreaktionen zum Einsatz gelangen, durchgeführt. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben. Ein anderes Konzept als Festbettreaktoren sind beispielsweise Reaktoren, in denen der Ionenaustauscher oder Zeolith suspendiert in einer flüssigen Phase vorliegt.

Als Reaktoren können bei der Isomerisierung Rohrreaktoren oder Rohrbündelreaktoren, insbesondere solche mit inneren Rohrdurchmessern von 10 bis 60 mm, verwendet werden. Das Verhältnis von Länge zu Durchmesser der Katalysatorschüttung kann dabei variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht und der Wärmeübergang an das Kühlmedium gezielt beeinflusst werden.

Die Kühlung der Rohre des Reaktors, sei es Rohrreaktor oder Rohrbündelreaktor, kann über ein Kühlmedium (z.B. Kühlwasser oder zur Wärmeintegration ein wärmeaufnehmendes Prozessfluid) über den Mantelraum des Reaktors oder einen Wärmeübertrager in einem externen Recycle erfolgen. Insbesondere bei Einsatz von flüssigen Heizmedien wird dabei die Mantelseite konstruktiv so ausgeführt, dass ein möglichst homogener Temperaturgradient an allen Rohren anliegt. Die hierfür notwendigen technischen Maßnahmen sind dem Fachmann bekannt und in der Literatur beschrieben (Einbau von Umlenkblechen, Disc- an Donut-Bauweise, Einspeisung / Abfuhr des Wärmeträgers an verschiedenen Stellen des Reaktors usw.). Bevorzugt werden Reaktionsmedium und Wärmeträger im Gleichstrom, besonders bevorzugt von oben nach unten durch die Reaktorrohre bzw. den Reaktormantel geführt. Eine bevorzugte Ausführungsform ist beispielsweise in DE 10 2006 040 433 A1 beschrieben.

Die Isomerisierung von 2,4,4-Trimethylpent-2-en zu 2,4,4-Trimethylpent-1-en erfolgt exotherm, verläuft also unter Freisetzung von Energie, was zu einer Erwärmung der Reaktionsmischung führt. Zur Begrenzung des Temperaturanstiegs ist eine Verdünnung des Einsatzstroms, beispielsweise durch Rückführung von Produkt möglich.

Der oder die bei der Isomerisierung eingesetzten Reaktor(en) können adiabatisch, polytrop oder praktisch isotherm betrieben werden. Praktisch isotherm bedeutet, dass die Temperatur an einer beliebigen Stelle im Reaktor maximal um 10°C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren eine Kühlung vorzusehen. Reaktoren, die für einen polytropen oder praktisch isothermen Betrieb geeignet sind, sind beispielsweise die bereits erwähnten Rohrbündelreaktoren, aber auch Rührkessel- und Schlaufenreaktoren.

Das Verfahren kann bei eher milden Temperaturen durchgeführt werden. Die Isomerisierung wird vorzugsweise bei einer Temperatur von 25 bis 90 °C, vorzugsweise 30 bis 80 °C, besonders bevorzugt 35 bis 70 °C durchgeführt. Weiterhin kann die erfindungsgemäße Isomerisierung bei einem Druck gleich oder größer als der Dampfdruck des Einsatzstromgemisches und/oder des Reaktionsgemisches bei der jeweiligen Reaktionstemperatur durchgeführt werden, vorzugsweise bei einem Druck von mehr als 0 bar aber weniger als 40 bar. Weiterhin bevorzugt wird die Isomerisierung in der Flüssigphase durchgeführt. Es sollte klar sein, dass in diesem Fall Druck und Temperatur so zu wählen sind, dass der Einsatzstrom in der flüssigen Phase vorliegt bzw. vorliegen kann.

In dem oder den Reaktor(en) können bei der Isomerisierung auch verschiedene Katalysatoren auf Basis eines Zeoliths oder eines Ionenaustauscherharzes eingesetzt werden. So kann beispielsweise ein Gemisch von lonenaustauscherharzen unterschiedlicher Reaktivität eingesetzt werden. Ebenso ist es möglich, dass ein Reaktor Katalysatoren mit unterschiedlicher Aktivität enthält, die beispielsweise in Schichten angeordnet sind. Wird mehr als ein Reaktor verwendet, können die einzelnen Reaktoren mit dem gleichen oder unterschiedlichen Katalysator(en) auf Basis eines Zeoliths oder eines Ionenaustauscherharzes gefüllt sein.

Als heterogener Katalysator kann bei der Isomerisierung ein Katalysator auf Basis eines Zeoliths oder eines Ionenaustauscherharzes eingesetzt werden. Entsprechende Zeolithe und lonenaustauscherharze sind zahlreich im Handel verfügbar. Es hat sich gezeigt, dass der Katalysator auf Basis eines Zeoliths vorzugsweise ein Si : AI Verhältnis im Bereich von 40 : 1 bis 200 : 1 aufweist. Weiterhin hat sich gezeigt, dass als Katalysator auf Basis eines Ionenaustauscherharzes vorzugsweise der Styrol-Divinylbenzol-Typ als H-Form und in teilneutraliserter Form gut geeignet ist.

Ist der Katalysator ein Zeolith, haben sich einige Zeolithe als besonders vorteilhaft herausgestellt, beispielsweise beta- und gamma-Zeolithe. Der Zeolith wird daher vorzugsweise aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt.

Als lonenaustauscherharz können beispielsweise lonenaustauscherharze eingesetzt werden, solche, die durch die Sulfonierung von Phenol/Aldehyd-Kondensaten oder durch die Sulfonierung von Copolymeren von aromatischen Vinylverbindungen hergestellt werden. Beispiele für aromatische Vinylverbindungen zur Herstellung der Copolymere sind: Styrol, Vinyltoluol, Vinylnaphthalin, Vinylethylbenzol, Methylstyrol, Vinylchlorbenzol, Vinylxylol und Divinylbenzol. Besonders bevorzugt werden lonenaustauscherharze für die Isomerisierung eingesetzt, die durch die Sulfonierung von Copolymerenn, die durch Umsetzung von Styrol mit Divinylbenzol entstehen, hergestellt werden. Die lonenaustauscherharze können gelförmig, makroporös oder schwammförmig hergestellt werden. Die Eigenschaften dieser Harze, insbesondere spezifische Oberfläche, Porosität, Stabilität, Quellung oder Schrumpfung und Austauschkapazität, können bekanntermaßen durch den Herstellprozess variiert werden.

lonenaustauscherharze des bevorzugten Styrol-Divinylbenzol-Typs werden u. a. unter folgenden Handelsnamen verkauft: CT 151 und CT275 der Firma Purolite, Amberlyst^{®} 15, Amberlyst^{®} 35, Amberlite^{®} IR-120, Amberlite^{®} 200 der Firma Rohm&Haas, Dowex M-31 der Firma DOW, LEWATIT^{®} K 2621, LEWATIT^{®} K 2431 der Firma Lanxess.

Das Porenvolumen der als Katalysatoren einsetzbaren lonenaustauscherharze, insbesondere des bevorzugten Styrol-Divinylbenzol-Typs, beträgt vorzugsweise 0,3 bis 0,9 ml/g, besonders bevorzugt 0,5 bis 0,9 ml/g. Das Porenvolumen kann beispielsweise mittels adsorptiver Techniken ermittelt werden. Die Korngröße der lonenaustauscherharze beträgt bevorzugt van 0,3 mm bis 1,5 mm, besonders bevorzugt 0,5 mm bis 1,0 mm. Die Korngrößenverteilung kann enger oder weiter gewählt werden. So können beispielsweise lonenaustauscherharze mit sehr einheitlicher Korngröße (monodisperse Harze) eingesetzt werden.

Die als Katalysatoren bei der Isomerisierung einsetzbaren lonenaustauscherharze können als teilneutralisierte lonenaustauscherharze vorliegen. Dazu kann das lonenaustauscherharz mit Säuren oder Basen behandelt werden, wie es in der EP 1 360 160 B1 beschrieben wird.

Aus der optionalen Isomerisierung wird ein Isomerisierungsstrom erhalten, bei dem der Anteil des 2,4,4-Trimethylpent-2-ens geringer und der Anteil des 2,4,4-Trimethylpent-1-ens im Isomerisierungsstrom höher ist als im eingesetzten Reststrom. Der Isomerisierungsstrom kann zumindest teilweise zur Destillation in Schritt a geführt werden, vorzugsweise wird der Isomerisierungsstrom vollständig zu Destillation in Schritt a geführt.

Das Verfahren kann weiterhin das Zuführen eines Einsatzstroms umfassen, mit dem das Verfahren gespeist wird. Der Einsatzstrom, mit dem das Verfahren gespeist wird und der 2,4,4-Trimethylpent-2-en und 2,4,4-Trimethylpent-1-en enthält, kann zusätzlich zum Isomerisierungsstrom zur Destillationseinheit in Schritt a und/oder zusätzlich zum Reststrom zur Isomerisierung geleitet werden.

Dabei ist die flexible Zuführung des Einsatzstroms vorteilhaft. Dadurch kann das Verfahren nämlich an die jeweiligen Gegebenheiten angepasst werden, beispielweise in Abhängigkeit des eingesetzten Einsatzstroms. Liegen die Anteile von 2,4,4-Trimethylpent-2-en und 2,4,4-Trimethylpent-1-en im Einsatzstrom nah an der Gleichgewichtsverteilung, kann der Strom direkt zur Destillation geführt werden. Bei von der Gleichgewichtsverteilung abweichenden Anteilen von 2,4,4-Trimethylpent-2-en und 2,4,4-Trimethylpent-1-en im Einsatzstrom könnte der Strom zunächst zur Isomerisierung geführt werden. Der Einbau in bestehende Produktionsanlagen ist somit in Abhängigkeit von der Zusammensetzung des Einsatzstroms auf einfache Weise möglich.

Der aus der Destillation in Schritt a erhaltene Kopfstrom wird, wie erwähnt, dem nachfolgenden Schritt b der Hydroformylierung zugeführt und dort umgesetzt.

Die Di-isobutene, d. h. 2,4,4-Trimethylpent-2-en und 2,4,4-Trimethylpent-1-en, werden in Schritt b mit Synthesegas (Mischung aus Kohlenmonoxid (CO) und Wasserstoff (H₂)) zu einem Aldehyd umgesetzt. Die Anzahl der Kohlenstoffatome im Aldehyd erhöht sich dabei im Vergleich mit dem eingesetzten Di-isobuten um 1 Kohlenstoffatom. Aus den Di-isobutenen (8 Kohlenstoffatome) entsteht demnach ein Aldehyd mit 9 Kohlenstoffatomen, nämlich Trimethylhexanal.

Das Synthesegas für das erfindungsgemäße Verfahren kann in unterschiedlichen Mischungsverhältnissen von Kohlenmonoxid und Wasserstoff eingesetzt werden. Das molare Verhältnis zwischen Synthesegas und dem eingesetzten Kohlenwasserstoffstrom, der die zu hydroformylierende Olefine enthält, sollte zwischen 6:1 und 1:1, vorzugsweise zwischen 3:1 und 1:1, besonders bevorzug zwischen 2:1 und 1:1 liegen. Die Hydroformylierung kann optional in Anwesenheit eines dem Fachmann bekannten zusätzlichen Lösemittels durchgeführt wird, vorzugsweise wird jedoch kein zusätzliches Lösemittel verwendet, sondern das eingesetzte Olefin fungiert bei der Hydroformylierung als Lösemittel.

Das bei der Hydroformylierung einsetzbare homogene Katalysatorsystem kann Co oder Rh, vorzugsweise Rh, und optional einen phosphorhaltigen Liganden umfassen. Entsprechende Katalysatorsysteme sind dem Fachmann geläufig. Der Einsatz eines phosphorhaltigen Liganden ist bevorzugt. In einer besonders bevorzugten Ausführungsform umfasst oder besteht das homogene Katalysatorsystem aus Rh und einem phosphorhaltigen Liganden. Geeignete Liganden für die erfindungsgemäßen Katalysatorsysteme sind dem Fachmann bekannt. Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 100 bis 350 bar, weiterhin bevorzugt im Bereich von 175 bis 325 bar und besonders bevorzugt im Bereich von 200 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck.

Homogene katalysierte Hydroformylierungen können als Flüssigaustragsverfahren ("liquid recycle") oder als Gasaustragsverfahren ("gas recycle") betrieben werden. Beide Verfahrensvarianten sind dem Fachmann bekannt und in vielen Lehrbüchern beschrieben. Eine konkrete Auswahl eines solchen Verfahrens ist im Rahmen der vorliegenden Erfindung nicht notwendig, weil das Verfahren grundsätzlich auf beide Arten durchgeführt werden kann. Wichtig bei der homogenen Katalyse ist allenfalls noch die Abtrennung des Katalysatorsystems aus dem Reaktionsaustrag. Bei einem flüssigen Austrag ist dies beispielsweise über Flashverfahren oder Membrantrennung möglich. Bei einem gasförmigen Austrag beispielsweise mittels Kondensation und/oder Auswaschen. Auch dies ist dem Fachmann bekannt und darf keiner ausführlichen Erläuterung. Die weitere Aufarbeitung des Reaktionsaustrags, insbesondere die Abtrennung des Reaktionsprodukts, ist dem Fachmann ebenfalls geläufig und kann beispielsweise mittels eines thermischen Trennverfahrens wie der Destillation erfolgen. Thermische Trennung bzw. thermische Trennverfahren im Sinne der vorliegenden Erfindung meint ein Trennverfahren, bei dem die Trennung anhand des Siedepunktes erfolgt.

Durch die beschriebene Hydroformylierung in Schritt b wird ein flüssiges Produktgemisch erhalten, das zumindest den durch die Hydroformylierung gebildeten Aldehyd, das homogene Katalysatorsystem und nicht umgesetzte Di-isobutene 2,4,4-Trimethylpent-2-en und 2,4,4-Trimethylpent-1-en umfasst.

Das so erhaltene Produktgemisch wird dem nachfolgenden Schritt c zugeführt, um das homogene Katalysatorsystems aus dem flüssigen Produktgemisch abzutrennen. Vor dem Zuführen des Produktgemisch können bereits leichtsiedende Komponenten beispielsweise leichtsiedende Nebenprodukte abgetrennt werden, beispielsweise mittels thermischer Trennung (Flash, Destillation, o. ä.), wofür eine Entspannung des unter hohem Druck stehenden Produktgemischs notwendig sein kann.

In einer bevorzugten Ausführungsform der vorliegenden Erfindung erfolgt weiterhin eine Kühlung des Produktgemischs vor der Membrantrennung in Schritt c auf eine Temperatur zwischen 40 und 100 °C, vorzugsweise zwischen 50 und 95 °C, besonders bevorzugt zwischen 60 und 90°C. Dazu wird eine geeignete Kühlvorrichtung benötigt. Das Kühlen erfolgt insbesondere mit einem Austragskühler. Als zweckmäßig haben sich beispielsweise Rohrbündelwärmetauscher erwiesen, wobei das Reaktionsgemisch vorzugsweise durch die Rohe und das Kühlmedium vorzugsweise durch den Mantel des Wärmetauschers geführt wird.

Die Kühlung des Produktgemischs sorgt für eine Verringerung des Katalysatormetall-Einsatzfaktors. Das Problem ist, dass während einer Hydroformylierung und der nachfolgenden Abtrennung immer ein kleiner Teil des Metalls, insbesondere des Rhodiums auf verschiedene Weise verloren geht. Aufgrund der hohen Preise für die einzusetzenden Metalle, insbesondere des Metalls erhöht das die Prozesskosten, weil die Verluste durch Nachdosierung ausgeglichen werden müssen. Das erfindungsgemäße Kühlen hat nun jedoch den Effekt, dass der Einsatzfaktor sinkt, also weniger Katalysatormetall, insbesondere Rhodium verloren geht und deshalb weniger nachdosiert werden muss. Die Prozesskosten können also auf diese Weise erheblich gesenkt werden.

Die Abtrennung des homogenen Katalysatorsystems unter Erhalt des Rohproduktgemisches in Schritt c kann mithilfe verschiedener Trennverfahren erfolgen, beispielsweise mittels thermischer Trennung und/oder durch Membrantrennung. Entsprechende Verfahren sind dem Fachmann geläufig. Es ist bevorzugt, wenn zuerst eine thermische Trennung, beispielsweise eine Verdampfung und anschließend eine Membrantrennung erfolgt. Bei der Verdampfung gehen hauptsächlich Produktaldehyde (Trimethylhexanal) und nicht umgesetzten Di-isobutenen als Rohproduktgemisch über Kopf. Im Sumpf fällt eine Hochsiederphase an, die den homogenen Katalysator, Trimethylhexanal und eventuell entstandene Hochsieder enthält. Die Hochsiederphase kann dann einer Membrantrennung unterzogen werden, um mögliche Hochsieder auszuschleusen. Bei einer Membrantrennung fallen bekanntermaßen ein Retentat und ein Permeat an. Das Permeat kann einer weiteren Aufarbeitung zugeführt werden.

Das Retentat enthält dabei das homogene Katalysatorsystem. Es ist erfindungsgemäße bevorzugt, dass das Retentat zur Hydroformylierung in Schritt b bzw. zur Reaktionszone, wo die Hydroformylierung durchgeführt wird, zurückgeführt wird. So kann das Katalysatorsystem wiederverwendet werden. Bei der bevorzugt kontinuierlichen Durchführung des beanspruchten Verfahrens entsteht so ein Katalysatorkreislauf, wo wenn überhaupt nur geringfügige prozessbedinge Katalysatorverluste ausgeglichen werden müssen. Sofern der Di-isobutenstrom und das homogene Katalysatorsystem gemäß der bevorzugten Ausführungsform vor der Hydroformylierung in Schritt b insbesondere in einem geeigneten Mischbehälter vermischt werden wird, wird das Retentat zum Mischbehälter geführt.

Bei der Membrantrennung kann jedes geeignete Membranmaterial eingesetzt werden. Vorzugsweise wird bei der Membrantrennung nach der Verdampfung des erfindungsgemäßen Verfahrens ein OSN-Membranmaterial (OSN = Organic Solvent Nanofiltration) eingesetzt. Ein solches Membranmaterial besteht vorzugsweise zumindest aus einer trennaktiven Schicht (auch: aktiven Trennschicht) und einer Unterstruktur, worauf sich die trennaktive Schicht befindet. Bevorzugt besteht das erfindungsgemäße Membranmaterial zumindest aus einer trennaktiven Schicht und einer Unterstruktur.

Die Membrantrennung nach der Verdampfung wird vorzugsweise bei einer Temperatur im Bereich von 25 bis 100°C, weiterhin bevorzugt im Bereich von 30 bis 80°C und besonders bevorzugt im Bereich von 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird.

Der Transmembrandruck (TMP) liegt bei der Membrantrennung in Schritt c vorzugsweise im Bereich von 10 bis 60 bar, weiterhin bevorzugt im Bereich von 15 bis 55 bar, besonders bevorzugt im Bereich von 40 bis 50 bar. Der permeatseitige Druck kann dabei oberhalb des atmosphärischen Drucks liegen und vorzugsweise bis 15 bar, vorzugsweise 2 bis 7 bar betragen. Aus der Differenz von TMP und permeatseitigem Druck ergibt sich der retentatseitige Druck. In einer bevorzugten Ausführungsform sollte bei den Druckverhältnissen und insbesondere beim permeatseitigen Druck darauf geachtet werden, dass der Druck so eingestellt wird, dass eine Verdampfung nach dem Durchtritt durch die Membran vermieden wird. Ein Verdampfen könnte zu einer instabilen Fahrweise führen.

Im anschließenden Schritt d erfolgt die destillative Aufarbeitung des Rohproduktgemisches in mindestens einer Destillationskolonne zur Abtrennung der nicht umgesetzten Di-isobutene. Dabei wird ein Aldehydprodukt erhalten, welches die gebildeten Aldehyde enthält. Das Aldehydprodukt enthält die aus dem Di-isobuten gebildeten Aldehyde, also Trimethylhexanal.

Bei der destillativen Aufarbeitung des Rohproduktgemisches in Schritt d fallen die nicht umgesetzten Di-isobutene am Kopf der mindestens einen Destillationskolonne an. Aldehydprodukt fällt folglich im Sumpf der mindestens einen Destillationskolonne an. Der Kopfstrom, der die in der mindestens einen Destillationskolonne abgetrennten nicht umgesetzten Di-isobutene enthält, wird zur Hydroformylierung in Schritt b zurückgeführt. Ist eine Vermischung der eingesetzten Komponenten vor der Hydroformylierung vorhanden, wird der Kopfstrom natürlich zur Vermischung geführt. Dadurch ist ein kontinuierlicher Betrieb des erfindungsgemäßen Verfahrens unter höchstmöglicher Ausbeute möglich. Aus dem zurückgeführten Kopfstrom kann ein Purge abgezogen werden, um leichtsiedende Nebenprodukte aus dem Verfahren auszuschleusen.

Die destillative Aufarbeitung zur Abtrennung der nicht umgesetzten Di-isobutene in Schritt d kann in einer Destillationskolonne erfolgen. Denkbar wäre, dass die destillative Aufarbeitung zur Abtrennung der nicht umgesetzten Di-isobutene in Schritt d in mehreren Destillationskolonnen erfolgt. Dies würde aber einen deutlichen höheren apparativen Aufwand bedeuten. Bevorzugt ist deshalb, dass die destillative Aufarbeitung in Schritt d in einer einzigen Destillationskolonne erfolgt.

Der Druck in der Destillationskolonne bei der destillativen Aufarbeitung in Schritt d liegt vorzugsweise im Bereich von 0,3 bis 2 bar, weiterhin bevorzugt im Bereich von 0,4 bis 1 bar, besonders bevorzugt im Bereich 0,5 bis 0,7 bar beträgt. Die Temperatur im Sumpf der Destillationskolonne bei der destillativen Aufarbeitung in Schritt d liegt vorzugsweise im Bereich von 80 °C bis 160 °C. Die Temperatur am Kopf der Destillationskolonne bei der destillativen Aufarbeitung in Schritt d liegt vorzugsweise im Bereich von 30 bis 80 °C. Weiterhin ist es bevorzugt, dass das Rücklaufverhältnis in der Destillationskolonne zwischen 1 und 2 beträgt. Die Destillationskolonne für die Abtrennung in Schritt d umfasst vorzugsweise 10 bis 30 theoretische Stufen umfasst. Die Destillationskolonne kann dabei Hochleistungsstrukturpackungen enthalten. Entsprechende Hochleistungsstrukturpackungen sind dem Fachmann bekannt.

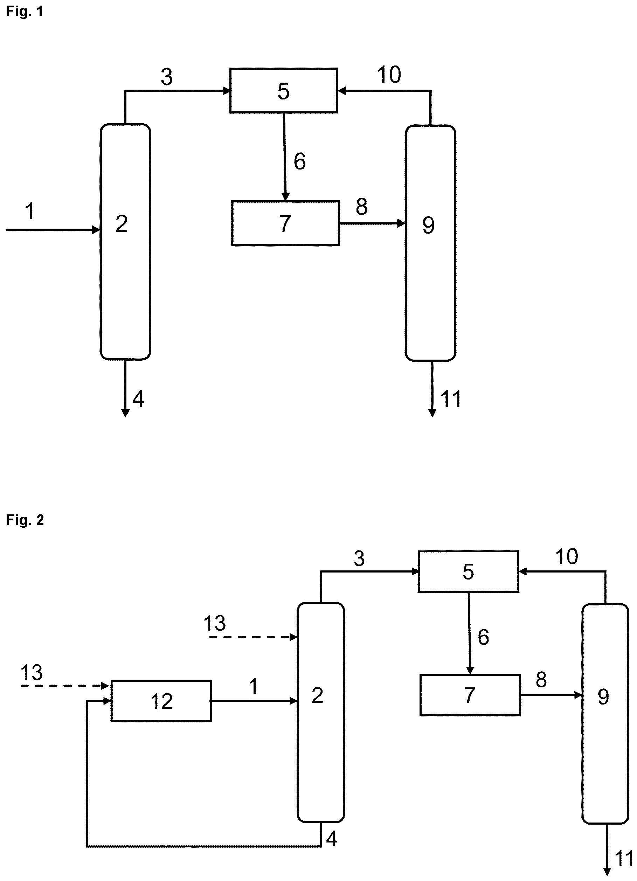

Die vorliegende Erfindung wird anhand der Abbildungen Fig. 1 und Fig. 2 näher erläutert. Die Zeichnungen zeigen spezielle Ausführungsformen und sollen den Erfindungsgegenstand nicht beschränken.

Fig. 1 zeigt eine Ausführungsform, bei der der Di-isobutenstrom (1) zur Destillationskolonne (2) in Schritt a geführt wird. Der Kopfstrom (3) ist gegenüber dem eingesetzten Di-isobutenstrom an 2,4,4-Trimethylpent-1-en angereichert. Bei der Destillation fällt außerdem der Reststrom (4) an, der gegenüber dem eingesetzten Di-isobutenstrom an 2,4,4-Trimethylpent-1-en abgereichert ist. Der Kopfstrom (3) wird nachfolgend zur Hydroformylierung (5) in Schritt b geführt, wodurch ein Produktgemisch (6) entsteht. Dieses Produktgemisch (6) wird durch die Katalysatorabtrennung (7) in Schritt c zum Rohproduktgemisch (8), welches in Schritt d einer Destillation (9) unterworfen wird. Dort fällt das Produkt (11) und das nicht umgesetzte 2,4,4-Trimethylpent-2-en und 2,4,4-Trimethylpent-1-en als Strom (10), welches zur Hydroformylierung (5) zurückgeführt wird.

Fig. 2 betrifft eine weiter Ausführungsform, bei der zusätzlich eine Isomerisierung (12) vorhanden ist. Der eingesetzte Di-isobutenstrom (13) kann dabei zur Isomerisierung und/oder zur Destillation (2) geführt werden. Dies wird durch die gestrichelten Linien angedeutet. Der isomerisierte Strom (1) wird dann der Zulauf zur Destillation (2). Die Isomerisierung wird zudem mit dem Reststrom (4) aus der Destillation gespeist, um dort 2,4,4-Trimethylpent-2-en zumindest teilweise in 2,4,4-Trimethylpent-1-en umzuwandeln.

## Patentansprüche

1. Verfahren zur Hydroformylierung von Di-isobuten, wobei das Verfahren zumindest die folgenden Schritte umfasst:
a. Zuführen eines Di-isobutenstroms, der 2,4,4-Trimethylpent-2-en und 2,4,4-Trimethylpent-1-en enthält, zu mindestens einer Destillationskolonne und Auftrennen des Di-isobutenstroms in mindestens einen gegenüber dem eingesetzten Di-isobutenstrom an 2,4,4-Trimethylpent-1-en angereicherten Kopfstrom, der mehr als 85 Gew.-% an 2,4,4-Trimethylpent-1-en enthält, und einen Reststrom, der gegenüber dem eingesetzten Di-isobutenstrom an 2,4,4-Trimethylpent-1-en abgereichert ist;
b. Zuführen des Kopfstroms zur Hydroformylierung, wodurch 2,4,4-Trimethylpent-2-en und 2,4,4-Trimethylpent-1-en mit Synthesegas in Gegenwart eines homogenen Katalysatorsystems, welches zumindest Co oder Rh und optional einen phosphorhaltigen Liganden umfasst, in einer Reaktionszone unter Erhalt eines flüssigen Produktgemisches umgesetzt werden, wobei das flüssige Produktgemisch zumindest die durch die Hydroformylierung gebildeten Aldehyde, das homogene Katalysatorsystem und nicht umgesetztes 2,4,4-Trimethylpent-2-en und 2,4,4-Trimethylpent-1-en umfasst;
c. Abtrennen des homogenen Katalysatorsystems aus dem flüssigen Produktgemisch unter Erhalt eines Rohproduktgemisches, welches zumindest die durch die Hydroformylierung gebildeten Aldehyde und nicht umgesetztes 2,4,4-Trimethylpent-2-en und 2,4,4-Trimethylpent-1-en umfasst;
d. Destillative Aufarbeitung des Rohproduktgemisches in mindestens einer Destillationskolonne zur Abtrennung von nicht umgesetzten 2,4,4-Trimethylpent-2-en und nicht umgesetzten 2,4,4-Trimethylpent-1-en, wobei das nicht umgesetzte 2,4,4-Trimethylpent-2-en und 2,4,4-Trimethylpent-1-en abgetrennt und zur Hydroformylierung in Schritt b zurückgeführt wird.

2. Verfahren nach einem der vorhergehenden Ansprüche, wobei der Reststrom in Schritt a) als Sumpfstrom oder als Seitenstrom an der mindestens einen Destillationskolonne abgenommen wird.

3. Verfahren nach einem der vorhergehenden Ansprüche, wobei der Reststrom zu einer Isomerisierung geführt wird, bei der 2,4,4-Trimethylpent-2-en unter Verwendung eines heterogenen Katalysators auf Basis eines Zeoliths oder eines Ionenaustauscherharzes zumindest teilweise zu 2,4,4-Trimethylpent-1-en isomerisiert wird.

4. Verfahren nach Anspruch 3, wobei aus der Isomerisierung ein Isomerisierungsstrom erhalten wird, in dem der Anteil des 2,4,4-Trimethylpent-2-ens geringer und der Anteil des 2,4,4-Trimethylpent-1- höher ist als im Reststrom.

5. Verfahren nach Anspruch 3 oder 4, wobei der Isomerisierungsstrom zumindest teilweise zur Destillation in Schritt a geführt wird.

6. Verfahren nach einem der Ansprüche 3 bis 5, wobei ein Einsatzstrom, mit dem das Verfahren gespeist wird und der 2,4,4-Trimethylpent-2-en und 2,4,4-Trimethylpent-1-en enthält, zusätzlich zum Isomerisierungsstrom zur Destillationseinheit in Schritt a und/oder zusätzlich zum Reststrom zur Isomerisierung geführt wird.

7. Verfahren nach einem der Ansprüche 3 bis 6, wobei die Isomerisierung bei einer Temperatur von 25 bis 90 °C, vorzugsweise 30 bis 80 °C, besonders bevorzugt 35 bis 70 °C durchgeführt wird.

8. Verfahren nach einem der Ansprüche 3 bis 7, wobei das Zeolith ein Si : AI Verhältnis im Bereich von 40 : 1 bis 200 : 1 aufweist.

9. Verfahren nach einem der Ansprüche 3 bis 8, wobei das Zeolith aus der Gruppe der beta- und gamma-Zeolithe ausgewählt wird.

10. Verfahren nach einem der Ansprüche 3 bis 7, wobei als lonenaustauscherharz solche eingesetzt werden, die durch die Sulfonierung von Phenol/Aldehyd-Kondensaten oder durch die Sulfonierung von Copolymeren von aromatischen Vinylverbindungen hergestellt werden.

11. Verfahren nach einem der vorhergehenden Ansprüche, wobei die Hydroformylierung in Schritt b bei einer Temperatur von 90 bis 250 °C, vorzugsweise von 120 bis 200 °C, besonders bevorzugt von 120 bis 170 °C durchgeführt wird.

12. Verfahren nach einem der vorhergehenden Ansprüche, wobei die Hydroformylierung in Schritt b bei dem Druck von 100 bis 350 bar, vorzugsweise 175 und 325 bar, besonders bevorzugt 200 bis 300 bar durchgeführt wird.

13. Verfahren nach einem der vorhergehenden Ansprüche, wobei die Abtrennung des homogenen Katalysatorsystems in Schritt c mittels thermischer Trennung und /oder Membrantrennung erfolgt.

14. Verfahren nach einem der vorhergehenden Ansprüche, wobei die Abtrennung des homogenen Katalysatorsystems in Schritt c mittels Verdampfung und anschließender Membrantrennung erfolgt.
